Europäisches Patentamt

European Patent Office

Office européen des brevets

(19)

(11) Veröffentlichungsnummer: **0 149 151**
**B1**

(12) **EUROPÄISCHE PATENTSCHRIFT**

(45) Veröffentlichungstag der Patentschrift:
09.08.89

(21) Anmeldenummer: 84115368.7

(22) Anmeldetag: 13.12.84

(51) Int. Cl.⁴: $B\ 01\ J\ 8/38//\ C02F11/02,$
$C12M1/40$

(54) Vorrichtung zum Beschleunigen der Stoffumsetzung zwischen zwei in einem Fliessbett reagierenden Medien.

(30) Priorität: 23.12.83 DE 3346861

(43) Veröffentlichungstag der Anmeldung:
24.07.85 Patentblatt 85/30

(45) Bekanntmachung des Hinweises auf die Patenterteilung: 09.08.89 Patentblatt 89/32

(84) Benannte Vertragsstaaten:
AT BE CH FR GB IT LI NL SE

(56) Entgegenhaltungen:
CH—A— 615 841
DE—A— 2 529 614
DE—A— 2 545 680
DE—A— 2 629 958
JP—A—54 114 474
JP—A—54 141 379
US—A— 4 242 450

(73) Patentinhaber: Krauss-Maffei Aktiengesellschaft
Krauss-Maffei-Strasse 2
D-8000 München 50 (DE)

(72) Erfinder: Alstetter, Franz, Dipl.-Ing.
Augustenfelderweg 1 B
D-8047 Karlsfeld (DE)
Erfinder: Hultsch, Günther, Ing.
Am Stichgartl 1
D-8042 Oberschleissheim (DE)

**Beschreibung**

Die Erfindung bezieht sich auf eine Vorrichtung zum Beschleunigen der Reaktion zwischen zwei Medien gemäß Oberbegriff des Anspruchs 1.

Aus der DE-OS 25 45 680 ist eine Vorrichtung bekannt, bei der zur Durchführung der Reaktion zwischen einem gasförmigen und einem festen Reaktionsmaterial das gasförmige Reaktionsmaterial das feste, aus Teilchen bestehende Reaktionsmaterial unter Bildung eines Fließbettes durchströmt, das, um die Relativgeschwindigkeit zwischen den Reaktionsmaterialien steigern zu können, einem Zentrifugalfeld ausgesetzt wird.

Aus der JP-54-114 474-A 1 und der JP-54-141 379-A 1 ist es bereits auch bekannt, ein Fließbett einem Zentrifugalfeld auszusetzen, in dem eine feste und auch eine flüssige Phase zur Reaktion gebracht werden können.

Aus der Literatur, Lueger, Lexikon der Verfahrenstechnik, Band 16, vierte Auflage, Seite 585 ist es ferner bekannt, die Relativgeschwindigkeit zwischen feinkörnigen Feststoffpartikeln und einem flüssigen Trägerstrom mittels einer Fließbettanordnung zu steigern. Insbesondere bei geringer Partikelgröße und teilweise geringer Dichtdifferenz sind jedoch die Archimedeszahlen so klein, daß der Stoffumsatz mit der Fließbettanordnung nur unwesentlich vergrößert werden kann, weil nach wie vor die benetzten Festkörper von einer Grenzschicht der Flüssigkeit umhüllt bleiben, innerhalb derer keine für einen guten Stoffaustausch ausreichende Relativgeschwindigkeit zwischen den beiden Medien erzielt werden kann. Der die Reaktionsgeschwindigkeit überwiegend bestimmende Mechanismus ist dann nur die Diffusion in der Grenzschicht.

Der Erfindung liegt daher die Aufgabe zugrunde, die chemische, biochemische, metallurgische oder katalytische Reaktion zwischen zwei Medien fester und flüssiger Phase durch Erhöhung der Relativgeschwindigkeit an den Phasen-Grenzflächen zu beschleunigen, wobei insbesondere auch die Stoffaustauschraten bei Medien mit geringer partikelgröße und/oder mit geringen Dichtunterschieden gesteigert werden sollen.

Diese Aufgabe wird durch die Merkmale aus dem Kennzeichen des Hauptanspruchs gelöst. Während sich mit der herkömmlichen Fließbettechnik eine Erhöhung der Archimedeszahlen lediglich um den Faktor 5 erzielen läßt, können die Archimedeszahlen nach der Erfindung proportional der Zentrifugalbeschleunigung, also z. B. mindestens um den Faktor 1 000 vergrößert werden. Damit ergibt sich der Vorteil, daß auch in Bereichen der Biotechnologie ein guter Stoffaustausch erreicht werden kann, so z. B. bei der Abwasserbehandlung mittels der sogenannten Fermentation, bei der auf Feststoffpartikeln aufgewachsene mikrobiologische Kulturen (Bakterien, Zellen, Enzyme etc.) den Stoffumsatz bewirken.

In einer bevorzugten Ausführungsform wird das einem Zentrifugalfeld aussetzbare Fließbett durch den Siebzylinder einer rotierend antreibbaren Siebtrommel gebildet, wobei Einrichtungen zur Erzeugung eines Druckgefälles vorgesehen sind, aufgrund dessen die Phase, die die andere Phase in der Schwebe hält, im wesentlichen in radialer Richtung durch den Siebzylinder tritt und die in der Schwebe zu haltende Phase durchströmt. Mit der zylindrischen Siebfläche und deren Durchströmungs-Druckverlust wird sichergestellt, daß über die gesamte Fließbettfläche gleiche Durchströmungsverhältnisse bestehen, wobei die Anordnung von Zufuhr- und Austrageinrichtungen keine technischen Schwierigkeiten beinhaltet und auch einen kontinuierlichen Betrieb der erfindungsgemäßen Fließbettanordnung ermöglicht.

Zur Beschleunigung der Reaktion zwischen zwei Medien, von denen die durchströmende phase eine geringere und die in der Schwebe zu haltende phase eine größere Dichte aufweisen, ist es zweckmäßig, die Siebtrommel gemäß Anspruch 3 zu gestalten, wobei das zur Durchströmung erforderliche Druckgefälle in einfacher Weise durch die radiale Verstellung des ersten Schälrohrs und/oder die Einstellung einer größeren Füllhöhe in der Ringkammer steuerbar ist.

Vorzugsweise ist der Reaktionsraum von einem zweiten, radial inneren Siebzylinder begrenzt, durch den die im Reaktionsraum stattfindende Fließbettströmung von durch das erste Schälrohr verursachten Strömungsstörungen freigehalten werden kann. Der radial innere Siebzylinder ist gemäß Anspruch 19 auch mit einem Filtermedium ausrüstbar, durch das sichergestellt wird, daß auch feinste Partikelchen im Reaktionsraum zurückgehalten werden können.

Vorteilhafterweise sind die Ringkammer und die Leitung gemäß Anspruch 6 als geschlossenes System ausgebildet, wodurch dem durchströmenden Medium neben dem hydrostatischen Druck der radialen Flüssigkeitssäule auch noch ein Vordruck aufgeprägt werden kann. Desgleichen kann auch zum Zweck der Gegenspülung des Filtermediums nach Anspruch 19 auch ein kurzzeitig wirkender Unterdruck erzeugt werden.

Zur Durchführung und Beschleunigung aerober Prozesse, wie sie z. B. beim biologisch wirkenden Abbau von Schadstoffen aus Abwässern gegeben sind, bietet sich die Möglichkeit, den beiden Medien gemäß den Ansprüchen 7 und 8 ein gasförmiges Reaktionsmedium wie z. B. Sauerstoff in feiner Verteilung zuzuführen.

Desgleichen ergibt sich die Möglichkeit in den Reaktionsraum weitere feste und/oder flüssige Reaktionsmedien kontinuierlich oder intervallweise einzugeben, wobei das zusätzliche Reaktionsmedium dem Reaktionsraum direkt über den Innenraum der Siebtrommel oder über die Zuführleitungen der festen und/oder flüssigen Phasen zuführbar ist.

Zur Beschleunigung der Reaktion zwischen zwei Medien, von denen die durchströmende Phase eine größere und die in der Schwebe zu haltende phase eine geringere Dichte aufweisen, ist es zweckmäßig, den Siebzylinder gemäß den Ansprüchen 10 bis 17 auszugestalten.

Eine besonders vorteilhafte Ausführungsform beinhaltet sowohl die Merkmale gemäß den Ansprüchen 3 bis 9 als auch die Merkmale gemäß den Ansprüchen 10 bis 17, wodurch mit lediglich einer, gemäß dem Anspruch 18 ausgestalteten Vorrichtung sämtliche feste und/oder flüssige Medien unabhängig von ihrer Dichtebeschaffenheit verarbeitet werden können.

Vorzugsweise sind auch die Vorrichtungen gemäß den Ansprüchen 10 bis 17 sowie 18 mit einem Filtermedium nach dem Anspruch 19 zum Zurückhalten der mit der durchströmenden phase mitgerissenen Feinstpartikel ausrüstbar.

Die Erfindung wird anhand von vier, in der Zeichnung dargestellten Ausführungsbeispielen erläutert.

Dabei zeigt

Fig. 1  den Längsschnitt durch eine Fließbett-Siebtrommel,

Fig. 2  den Längsschnitt durch einen Teilbereich einer Fließbett-Siebtrommel mit einer anderen Ausführungsform der Ringkammer,

Fig. 3  den Längsschnitt durch eine weitere Ausführungsform einer Fließbett-Siebtrommel und

Fig. 4  den Längsschnitt durch eine universellverwendbare Ausführungsform einer Fließbett-Siebtrommel.

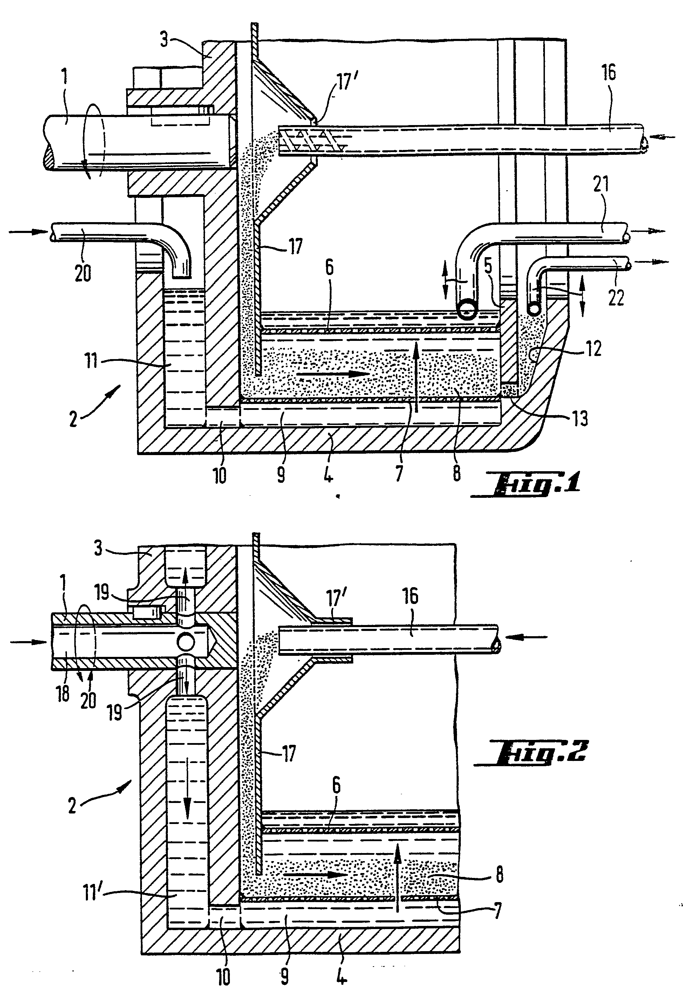

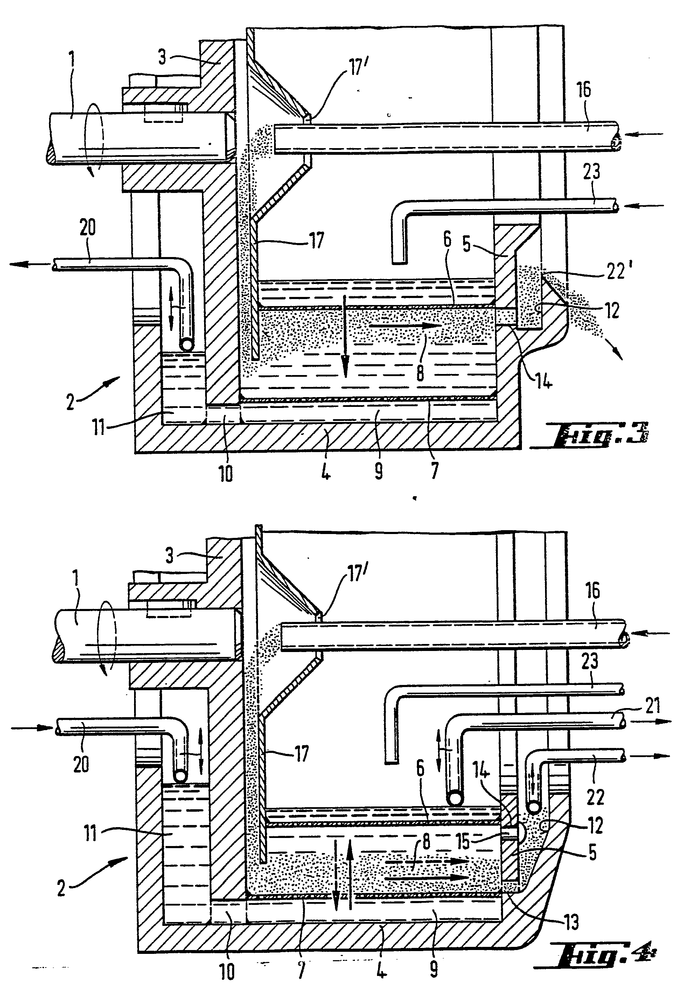

Nach den Fign. 1, 3 und 4 ist auf einer drehbar gelagerten Welle 1 eine Siebtrommel 2 befestigt, die sich im wesentlichen aus dem Trommelboden 3, der Trommelwand 4 und einem ringförmigen Bordring 5 zusammensetzt. Innerhalb der Siebtrommel 2 sind ein radial innerer und ein radial äußerer Siebzylinder 6 und 7 angeordnet, die einen Reaktionsraum 8 umschließen. Radial außerhalb des radial äußeren Siebzylinders 7 schließt sich ein Druckraum 9 an, der über eine Durchflußbohrung 10 mit einer axial an den Trommelboden 3 anschließenden Ringkammer 11 in Verbindung steht. Axial außerhalb des ringförmigen Bordrings 5 ist eine Abfluß-Ringkammer 12 angeordnet, die in der Ausführungsform nach Fig. 1 mit dem Reaktionsraum 8 über eine radial äußere Abflußbohrung 13 und in der Ausführungsform nach Fig. 3 über eine radial innere Abflußbohrung 14 mit dem Reaktionsraum 8 in Verbindung steht. Die Ausführungsform nach Fig. 4 hat im ringförmigen Bordring 5 sowohl die radial innere als auch die radial äußere Abflußbohrung 13 und 14 angeordnet, die wechselweise mit einem Verschlußstopfen 15 verschließbar sind.

In Fig. 4 ist die radial innere Abflußbohrung 14 verschlossen, so daß die dargestellte Siebtrommel 2 funktionell der Ausführungsform nach Fig. 1 entspricht.

In den Innenraum der Siebtrommel 2 nach den Figuren 1 bis 4 ragt ein drehfestes Zentral-Füllrohr 16, das in eine zentrale Öffnung 17' einer Leitscheibe 17 mündet, die im parallelen Abstand zum Trommelboden 3 an diesem befestigt ist. Die Leitscheibe 17 bildet an ihrem äußeren Rand mit dem Trommelboden 3 eine Ringöffnung, die in den Reaktionsraum 8 führt. Das Zentral-Füllrohr 16 kann ggf. mit einer Förderschnecke ausgestaltet sein.

Nach der Fig. 2 ist die Welle 1 mit einer Bohrung 18 versehen, von der aus radial gerichtete und in eine geschlossene Ringkammer 11' führende Kanäle 19 angeordnet sind.

Nach Fig. 1 mündet in die Ringkammer 11 eine Zulaufleitung 20 und in den Siebtrommelinnenraum ragt ein erstes Schälrohr 21, dessen Schälöffnung in radialer Richtung, ggf. intervallmäßig verstellbar ist. In die Abfluß-Ringkammer 12 ragt ein zweites Schälrohr 22, das ebenfalls in radialer Richtung verstellbar angeordnet sein kann.

Die Ausführungsformen nach den Figuren 1 und 2 sind zum Beschleunigen der Reaktion zwischen zwei Medien geeignet, von denen eine durchströmende Phase eine geringere und eine in einem Zentrifugalfeld auf einem Fließbett in der Schwebe zu haltende Phase eine größere Dichte aufweisen.

Das Fließbett wird gemäß der Ausführungsform nach Fig. 1 dadurch gebildet, daß die Phase mit geringerer Dichte über die Leitung 20 in die Ringkammer 11 geleitet wird, in der sich eine Flüssigkeitssäule radialer Höhe einstellt. Die Phase mit geringerer Dichte gelangt über die Durchflußbohrung 10 weiter in den Druckraum 9, von wo aus die Phase geringerer Dichte in einem radial nach innen gerichteten Strom durch den radial äußeren Siebzylinder 7 tritt und auf die Phase mit höherer Dichte trifft und diese derart durchströmt, daß sie in der Schwebe gehalten wird. Die Phase höherer Dichte gelangt über das Zentral-Füllrohr 16 und den vom Trommelboden 3 und der Leitscheibe 17 gebildeten Zwischenraum in den Reaktionsraum 8 und verteilt sich in einer schweben= den Fließbewegung über die radial äußere Siebfläche 7. Die die Phase höherer Dichte radial nach innen durchströmende Phase geringerer Dichte wird nach der Umsetzung im Reaktionsraum 8 in radialer Höhe des ringförmigen Bordrings 5 von einem ersten Schälrohr 21 entnommen und nach außen abgeführt. Die von der Phase geringerer Dichte in der Schwebe gehaltene feste Phase höherer Dichte wandert in axialer Richtung zu der radial äußeren Abflußbohrung 13 und gelangt durch diese in die Abfluß-Ringkammer 12, von wo aus sie vom zweiten Schälrohr 22 ausgetragen wird. Um eine Störung der im Reaktionsraum 8 sich einstellenden Fließbettströmung durch das Eintauchen des ersten Schälrohres 21 zu vermeiden, dient der radial innere Siebzylinder 6 als Abschirmeinrichtung. Zum Zurückhalten von durch die Fließbettströmung mitgerissenen Feinstpartikeln kann am inneren Siebzylinder ein Filtermedium (nicht dargestellt) angeordnet sein.

Der Schwebezustand der festen Phase höherer Dichte wird dadurch erreicht, daß in Abhängigkeit der Dichtewerte der beiden Medien und der Drehzahl der Siebtrommel 2 ein bestimmtes hydrostatisches Druckgefälle zwischen Ringkammer 11 und Reaktionsraum 8 eingestellt wird. Dieses Druckgefälle ist durch die Zuflußmenge und die radiale Höheneinstellung des ersten Schälrohres 21 bestimmbar.

Das den Schwebezustand herbeiführende Druckgefälle ist gemäß der Ausführungsvariante nach Fig. 2 auch dadurch erreichbar, daß die Ringkammer 11 als geschlossenes System ausgebildet ist, dem über eine Bohrung 18 und radial gerichtete Kanäle 19 die Phase geringerer Dichte unter Druck zuführbar ist. Zur Druckerhöhung trägt auch die Tatsache bei, daß sich in der bis zur Welle 1 erstreckenden Ringkammer 11 durch die höhere radiale Flüssigkeitssäule auch ein höherer hydrostatischer Druck einstellen kann.

Die Ausführungsform nach Fig. 3 ist zum Beschleunigen der Reaktion zwischen zwei Medien geeignet, von denen eine durchströmende Phase eine höhere und eine in einem Zentrifugalfeld in der Schwebe zu haltende Phase eine geringere Dichte aufweisen.

Das Fließbett wird gemäß der Ausführungsform nach Fig. 3 dadurch gebildet, daß die phase mit höherer Dichte über ein Füllrohr 23 in den Innenraum der Siebtrommel 2 eingefüllt wird und in einer radial nach außen gerichteten Strömung durch den radial inneren Siebzylinder 6 tritt und auf die feste Phase mit geringerer Dichte trifft und diese derart durchströmt, daß sie über dem radial inneren Siebzylinder 6 in der Schwebe gehalten wird. Die Phase geringerer Dichte gelangt dabei über das Zentral-Füllrohr 16 und den vom Trommelboden 3 und der Leitscheibe 17 gebildeten Zwischenraum in den Reaktionsraum 8 und verteilt sich in einer schwebenden Fließbewegung über den radial inneren Siebzylinder 6. Die die Phase geringerer Dichte radial nach außen durchströmende Phase höherer Dichte gelangt in den von der Trommelwand 4 begrenzten Druckraum 9, von wo sie über die Durchflußbohrung 10 in die Ringkammer 11 strömt. Die Füllhöhe in dieser Ringkammer 11 wird durch die Leitung 20 bestimmt, die, einem Schälrohr gleichwirkend, die Phase höherer Dichte in einer radialen Höhe entnimmt, bei der sich zwischen dem Reaktionsraum 8 und der Ringkammer 11 ein hydrostatisches Druckgefälle einstellt, das die radial nach außen gerichtete Strömung der Phase höherer Dichte bewirkt.

Der Austrag der Phase geringerer Dichte, die vom Fließbett über die radial innere Abflußbohrung 14 in die Abfluß-Ringkammer 12 gefördert wird, kann über ein radial und ggf. intermittierend verstellbares zweites Schälrohr 22 (Fig. 1) oder, wie in Fig. 3 dargestellt, über eine Abwurfkante 22′ erfolgen, da die Phase geringerer Dichte in der Abfluß-Ringkammer 12 aufschwimmt und sich somit selbsttätig über die Abwurfkante fördert.

Die radial nach außen gerichtete Strömung der Phase höherer Dichte kann gemäß der Ausführungsform nach Fig. 2 auch dadurch erzielt bzw. unterstützt werden, daß der Zentrifugenraum als geschlossenes System ausgebildet wird und mit Überdruck beaufschlagt wird. Die flüssige Phase wird dabei in den unter Normaldruck stehenden Raum außerhalb der Zentrifuge transportiert.

Zum Zurückhalten von infolge der Fließbettströmung mitgerissenen Feinstpartikeln kann ein radial äußerer Siebzylinder 7 angeordnet sein, der ggf. noch mit einem Filtermedium belegt sein kann.

Bei der Ausführungsform nach Fig. 4, die eine Kombination der Ausführungsformen nach den Figuren 1 und 3 darstellt, ist die in die Ringkammer 11 ragende Zulaufleitung 20 auch als Schälrohr mit in radialer Richtung einstellbarer Schälöffnung verwendbar. In den Innenraum der Siebtrommel 2 ragen sowohl ein der Ausführungsform nach Fig. 3 entsprechendes Füllrohr 23 als auch ein der Ausführungsform nach Fig. 1 entsprechendes erstes Schälrohr 21. Mit der geöffneten radial äußeren Abflußbohrung 13 ist die Fließbett-Siebtrommel wie die Ausführungsform nach Fig. 1 betreibbar, so daß die Leitung 20 als Zulaufleitung für die Phase mit geringerer Dichte und das erste Schälrohr 21 für deren Abführung nach Durchströmung des Reaktionsraumes 8 dient, wobei das Füllrohr 23 außer Betrieb bleibt. Die Fließrichtung der beiden sich durchströmenden Medien unterschiedlicher Dichte ist mit den Pfeilen mit durchgehenden Linien dargestellt.

Wird anstelle der radial inneren Abflußbohrung 14 die radial äußere Abflußbohrung 13 verschlossen, ist die Fließbett-Siebtrommel wie die Ausführungsform nach Fig. 3 zu betreiben, wobei die Leitung 20 als Schälrohr benutzt wird und anstelle des ersten Schälrohres 21 das Füllrohr 23 verwendet wird. Die Fließrichtung der beiden sich durchströmenden Medien unterschiedlicher Dichte ist dabei mit den Pfeilen mit unterbrochenen Linien dargestellt.

Wie bei den Ausführungsformen nach den Figuren 1 und 3 kann auch die Ausführungsform nach Fig. 4 mit einer geschlossenen Ringkammer gemäß Fig. 2 ausgestattet sein, der über die Bohrung 18 und die Kanäle 19 das Medium entweder unter Druck zuführbar oder mittels Unterdruck abführbar ist.

Über die Zuführleitungen sämtlicher Ausführungsformen, wie dem Zentral-Füllrohr 16, der Bohrung 18 mit den Kanälen 19, der als Zulaufleitung dienenden Leitung 20 sowie dem Füllrohr 23 sind dem Reaktionsraum 8 weitere Reaktionspartner in gasförmiger, flüssiger oder fester Form zuführbar. So kann z.B. für die Durchführung und Beschleunigung aerober Prozesse, wie sie beim biologischen Abbau von Schadstoffen aus Abwässern stattfinden, Luft fein verteilt durch die Bohrung 18 eingeführt und durch die radial äußere Siebfläche 7 zum Reaktionsraum 8 transportiert werden. Desgleichen können über das Zentral-Füllrohr 16 und die Leitscheibe 17 z.B. katalytisch wirkende Feststoffe in den Reaktionsraum eingetragen werden. Grundsätzlich ist jede Art von zusätzlichen Reaktionspartnern auch durch gesonderte Leitungen in den zentralen Innenraum der Fließbett-Siebtrommel oder in die Ringkammer 11 einleitbar.

In der Regel wird die in der Schwebe zu haltende Phase aus feinkörnigen Feststoffteilchen bestehen, die von der durchströmenden Phase, der Reaktionsflüssigkeit in einem Fließbett bzw. einer Wirbelschicht in der Schwebe gehalten werden.

Grundsätzlich kann ein Medium, das gegenüber dem anderen Medium zwar eine höhere Dichte aufweist, aufgrund von Gasbildung oder Gasbindung dennoch spezifisch leichter werden, so daß es zu Flotation neigt. Hierfür eignet sich vorzugsweise die Ausführungsform nach Fig. 3, bei der die zu Flotation neigende Phase wie eine Phase geringerer Dichte zu behandeln ist.

## Patentansprüche

1. Vorrichtung zum Beschleunigen der Reaktion zwischen einer festen und einer flüssigen Phase unterschiedlicher Dichte in einem Zentrifugal-Fließbett, wobei ein Reaktionsraum von einem äußeren Siebzylinder sowie an seinen Stirnseiten von je einem Trommelboden begrenzt ist, der Reaktionsraum im Abstand von einem Außenmantel umgeben ist und auf einer drehbar gelagerten Welle befestigt ist, dadurch gekennzeichnet, daß der Reaktionsraum (8) radial nach innen von einem inneren Siebzylinder (6) begrenzt ist, einer der Trommelböden nur als ringförmige Wand (5) ausgebildet ist, der vom Außenmantel (4) umgebene Raum in mehrere Abschnitte unterteilt ist, wobei an einer Stirnseite des Reaktionsraumes (8) eine Ringkammer (11), sowie der ringförmigen Wand (5) benachbart eine Abflußringkammer (12), und radial nach außen an den äußeren Siebzylinder (7) des Reaktionsraumes (8) ein Druckraum (9) angeordnet sind, außerdem die Ringkammer (11) mit dem Druckraum (9) über eine Durchflußbohrung (10) und der Reaktionsraum (8) mit der Abflußringkammer (12) über eine Abflußbohrung (13) in Verbindung stehen, zur Beschickung des Reaktionsraumes (8) ein Zentral-Füllrohr (16) vorgesehen ist, ferner eine Leitung (20) in die Ringkammer (11) mündet, wobei für Reaktionen, bei denen die durchströmende Phase eine geringere und die in der Schwebe zu haltende Phase eine größere Dichte aufweisen, ein Rohr (21) zur Entnahme der Phase geringerer Dichte in dem Reaktionsraum (8) und ein weiteres Rohr (22) zur Entnahme der Phase größerer Dichte aus der Abflußringkammer (12) vorgesehen sind.

2. Vorrichtung nach Anspruch 1, dadurch gekennzeichnet, daß der Reaktionsraum (8) von einem zweiten, unterhalb der überlaufkante des ringförmigen Bordrings (5) angeordneten radial inneren Siebzylinder (6) begrenzt ist.

3. Vorrichtung nach den Ansprüchen 1 und 2, dadurch gekennzeichnet, daß die Zufuhr der Phase größerer Dichte in den zwischen dem radial äußeren (7) und dem radial inneren Siebzylinder (6) gelegenen Reaktionsraum (8) über eine im Parallelabstand zum Trommelboden (3) angeordnete Leitscheibe (17) mit einer zentral in dieser ausgebildeten öffnung (17') erfolgt, in die das Zentral-Füllrohr (16) einmündet.

4. Vorrichtung nach den Ansprüchen 1 bis 3, dadurch gekennzeichnet, daß die Ringkammer (11) und die Zulaufleitung (20) ein geschlossenes System darstellen, in dem die Zulaufleitung von einer in der Welle (1) angeordneten Bohrung (18) und von radialen, zu der Ringkammer (11) geführten Kanälen (19) gebildet wird, wobei die Bohrung (18) der Welle (1) über eine Drehkupplung an eine Druckquelle anschließbar ist.

5. Verfahren zum Betrieb der Vorrichtung nach den Ansprüchen 1 bis 4, dadurch gekennzeichnet, daß den beiden Medien ein gasförmiges Reaktionsmedium, beispielsweise in feiner Verteilung zugeführt wird.

6. Verfahren zum Betrieb der Vorrichtung nach den Ansprüchen 3 und 5, dadurch gekennzeichnet, daß das gasförmige Reaktionsmedium in die Ringkammer (11) und über die Durchflußbohrungen (10) in den Druckraum (9) eingeleitet wird.

7. Vorrichtung nach den Ansprüchen 1 bis 6, dadurch gekennzeichnet, daß der Siebtrommel (2) radial innerhalb des radial inneren Siebzylinders (6) über eine Zuführleitung ein festes oder flüssiges, ggf. mit einem Reaktionsgas angereichertes Reaktionsmedium zuführbar ist.

8. Vorrichtung zum Beschleunigen der Reaktion zwischen einer festen und einer flüssigen Phase unterschiedlicher Dichte in einem Fließbett, das einem Zentrifugalfeld ausgesetzt ist, wobei ein Reaktionsraum von einem äußeren Siebzylinder sowie an seinen Stirnseiten von je einem Trommelboden begrenzt ist, der Reaktionsraum im Abstand von einem Außenmantel umgeben ist und auf einer drehbar gelagerten Welle befestigt ist, dadurch gekennzeichnet, daß der Reaktionsraum (8) radial nach innen von einem inneren Siebzylinder (6) begrenzt ist, einer der Trommelböden nur als ringförmige Wand (5) ausgebildet ist, der vom Außenmantel (4) umgebene Raum in mehrere Abschnitte unterteilt ist, wobei an einer Stirnseite des Reaktionsraumes (8) eine Ringkammer (11), sowie der ringförmigen Wand (5) benachbart eine Abflußringkammer (12), und radial nach außen an den äußeren Siebzylinder (7) des Reaktionsraumes (8) ein Druckraum (9) angeordnet sind, außerdem die Ringkammer (11) mit dem Druckraum (9) über eine Durchflußbohrung (10) und der Reaktionsraum (8) mit der Abflußringkammer (12) über eine Abflußbohrung (13) in Verbindung stehen, zur Beschickung des Reaktionsraumes (8) ein Zentral-Füllrohr (16) vorgesehen ist, ferner eine Leitung (20) in die Ringkammer (11) mündet, wobei für Reaktionen zwischen zwei Medien, von denen die durchströmende Phase eine größere und die in der Schwebe zu haltende Phase eine geringere Dichte aufweisen, zur Beschickung des Reaktionsraumes (8) mit der Phase größerer Dichte ein in den Innenraum der Vorrichtung ragendes Füllrohr (23) vorgesehen ist.

9. Vorrichtung nach Anspruch 8, dadurch gekennzeichnet, daß der Reaktionsraum (8) radial innerhalb der im Trommelboden (3) angeordneten Durchflußbohrung (10) einen radial äußeren Siebzylinder (7) aufweist, der im wesentlichen den Druckraum (9) und den Reaktionsraum (8) voneinander trennt.

10. Vorrichtung nach den Ansprüchen 7 und 9, dadurch gekennzeichnet, daß die Leitung (20) zur Abführung der Reaktionsflüssigkeit bzw. der Phase mit größerer Dichte aus der Ringkammer (11) als Schälrohr ausgebildet ist, durch dessen radiale Verstellbarkeit das zur Durchströmung des Reaktionsraumes (8) in radial nach außen gerichteter Strömung erforderliche Druckgefälle beliebig, ggf. intermittierend einstellbar ist.

11. Vorrichtung nach den Ansprüchen 8 und 9, dadurch gekennzeichnet, daß die Ringkammer (11') ein geschlossenes System darstellt, in dem die Leitung von einer in der Welle (1) angeordneten Bohrung (18) und von radialen, aus der Ringkammer (11) führenden Kanälen (19) gebildet wird, und der Zentrifugenraum ebenfalls ein geschlossenes System darstellt, das mit Überdruck beaufschlagbar ist.

12. Vorrichtung nach Anspruch 11, dadurch gekennzeichnet, daß die Bohrung (18) der Welle (1) zwecks Gegenspülung des radial äußeren Siebzylinders (7) über eine Drehkupplung an eine Druckquelle anschließbar ist.

13. Verfahren zum Betrieb der Vorrichtung nach den Ansprüchen 8 bis 12, dadurch gekennzeichnet, daß den beiden Medien ein gasförmiges Reaktionsmedium in feiner Verteilung zugeführt wird.

14. Verfahren zum Betrieb der Vorrichtung nach Anspruch 13, dadurch gekennzeichnet, daß das gasförmige Reaktionsmedium in das Füllrohr (23) eingeleitet wird.

15. Vorrichtung nach Anspruch 13, dadurch gekennzeichnet, daß das gasförmige Reaktionsmedium über am Umfang der Trommelwand (4) verteilte Gasinjektionsdüsen zur Beaufschlagung des Reaktionsraums (8) in einem radial nach innen gerichteten Gegenstrom zur Strömung der Phase mit größerer Dichte zuführbar ist.

16. Vorrichtung nach den Ansprüchen 1 bis 15, dadurch gekennzeichnet, daß an der Siebtrommel (2) der ringförmige Bordring (5) sowohl die radial äußere als auch die radial innere Abflußbohrung (13, 14) aufweist, wobei, gemäß den Vorrichtungen nach den Ansprüchen 3 bis 9, entweder die Leitung (20) als Zuführungsleitung, das Zentral-Füllrohr (16), das zweite Schälrohr (22) und ggf. die im Abstand zum Trommelboden (3) angeordnete Leitscheibe (17) an der Siebtrommel in Betrieb setzbar sind und die radial innere Abflußbohrung (14) geschlossen und die radial äußere Abflußbohrung (13) geöffnet ist, oder, gemäß den Vorrichtungen nach den Ansprüchen 10 bis 17, die Leitung (20) als Schälrohr, das Füllrohr (23), das Zentral-Füllrohr (16) mit Leitscheibe (17) und das zweite Schälrohr (22) an der Siebtrommel (2) in Betrieb setzbar sind und die radial innere Abflußbohrung (14) geöffnet und die radial äußere Abflußbohrung (13) geschlossen ist.

17. Vorrichtung nach den Ansprüchen 1 bis 16, dadurch gekennzeichnet, daß zum Zurückhalten der mit der durchströmenden Phase mitgerissenen Feinstpartikel der in der Schwebe gehaltenen Phase ein Filtermedium angeordnet ist.

## Claims

1. Apparatus for accelerating the reaction between a solid and a fluid phase of different densities in a centrifugal fluidised bed, where a reaction chamber is limited by an external sieve cylinder and at each of its ends by a drum floor, the reaction chamber is surrounded at a distance by an external jacket and secured on a rotatably mounted shaft, characterised in that the reaction chamber (8) is radially inwardly limited by an inner sieve cylinder (6), one of the drum floors is formed only as annular wall (5), the chamber surrounded by the outer jacket (4) is divided into a plurality of sections, while an annular chamber (11) is arranged on one end of the reaction chamber (8), an outflow annular chamber (12) is arranged adjacent to the annular wall (5) and a pressure chamber (9) is arranged radially outwards on the outer sieve cylinder (7) of the reaction chamber (8), moreover the annular chamber (11) is in communication with the pressure chamber (9) through a passage bore (10) and the reaction chamber (8) is in communication with the outflow annular chamber (12) through an outflow bore (13), for the charging of the reaction chamber (8) a central filler pipe (16) is provided, further a conduit (20) opens into the annular chamber (11), while for reactions in which the through-flowing phase has a lower density and the phase to be kept in suspension has a higher density, a pipe (21) for withdrawal of the phase of lower density is provided in the reaction chamber (8) and a further pipe (22) is provided for the withdrawal of the phase of higher density from the outflow annular chamber (12).

2. Apparatus according to Claim 1, characterised in that the reaction chamber (8) is limited by a second radially inner sieve cylinder (6) arranged below the overflow edge of the annular rim ring (5).

3. Apparatus according to Claims 1 and 2, characterised in that the feed of the phase of higher density into the reaction chamber (8) placed between the radially outer sieve cylinder (7) and the radially inner sieve cylinder (6) takes place by way of a guide disc (17) arranged with parallel spacing from the drum floor (3) with an opening (17') formed centrally therein, into which the central filler pipe (16) opens.

4. Apparatus according to Claims 1 to 3, characterised in that the annular chamber (11) and the supply conduit (20) constitute a closed system in which the supply conduit is formed by a bore (18) arranged in the shaft (1) and by radial passages conducted to the annular chamber (11), while the bore (18) of the shaft (1) is connectable to a pressure source by way of a rotary coupling.

5. Process for the operation of the apparatus according to Claims 1 to 4, characterised in that a reaction medium in gaseous form is supplied, for example in fine distribution, to the two media.

6. Process for the operation of the apparatus according to Claims 3 and 5, characterised in that the gaseous reaction medium is introduced into the annular chamber (11) and through the passage bores (10) into the pressure chamber (9).

7. Apparatus according to Claims 1 to 6, characterised in that a solid or fluid reaction medium, which may be enriched with a reaction gas, can be supplied through a supply conduit to the sieve drum (2) radially within the radially inner sieve cylinder (6).

8. Apparatus for accelerating the reaction between a solid and a fluid phase of different densities in a fluidised bed which is exposed to a centrifugal field, where a reaction chamber is limited by an external sieve cylinder and at each of its ends by a drum floor, the reaction chamber is surrounded at a distance by an external jacket and secured on a rotatably mounted shaft, characterised in that the reaction chamber (8) is radially inwardly limited by an inner sieve cylinder (6), one of the drum floors is formed only as annular wall (5), the chamber surrounded by the outer jacket (4) is divided into a plurality of sections, while an annular chamber (11) is arranged on one end of the reaction chamber (8), an outflow annular chamber (12) is arranged adjacent to the annular wall (5) and a pressure chamber (9) is arranged radially outwards on the outer sieve cylinder (7) of the reaction chamber (8), moreover the annular chamber (11) is in communication with the pressure chamber (9) through a passage bore (10) and the reaction chamber (8) is in communication with the outflow annular chamber (12) through an outflow bore (13), for the charging of the reaction chamber (8) a central filler pipe (16) is provided, further a conduit (20) opens into the annular chamber (11), while for reactions between two media, of which the throughflowing phase has a higher density and the phase to be kept in suspension has a lower density, a filler pipe (23) extending into the interior of the apparatus is provided for charging the reaction chamber (8) with the phase of higher density.

9. Apparatus according to Claim 8, characterised in that the reaction chamber (8) comprises, radially within the passage bore (10) arranged in the drum floor (3), a radially outer sieve cylinder (7) which essentially separates the pressure chamber (9) and the reaction chamber (8) from one another.

10. Apparatus according to Claims 7 and 9, characterised in that the conduit (20) for conducting away the reaction fluid or the phase of higher density from the annular chamber (11) is formed as skimmer pipe by the radial adjustability of which the pressure drop necessary for the flow through the reaction chamber (8) in radially outwardly directed flow is adjustable as desired, possibly intermittently.

11. Apparatus according to Claims 8 and 9, characterised in that the annular chamber (11') constitutes a closed system in which the conduit is formed by a bore (18) arranged in the shaft (1) and by radial passages (19) leading out of the annular chamber (11), and the centrifuge chamber likewise constitutes a closed system which can be charged with excess pressure.

12. Apparatus according to Claim 11, characterised in that the bore (18) of the shaft (1) is connectable to a pressure source through a rotary coupling, for the purpose of counter-flushing of the radially outer sieve cylinder (7).

13. Process for the operation of the apparatus according to Claims 8 to 12, characterised in that a gaseous reaction medium in fine distribution is fed to the two media.

14. Process for the operation of the apparatus according to Claim 13, characterised in that the gaseous reaction medium is introduced into the filler pipe (23).

15. Apparatus according to Claim 13, characterised in that the gaseous reaction medium can be supplied by way of gas injection nozzles distributed on the circumference of the drum wall (4), for the charging of the reaction chamber (8), in a radially inwardly directed counter-current to the flow of the phase with higher density.

16. Apparatus according to Claims 1 to 15, characterised in that on the sieve drum (2) the annular rim ring (5) comprises both the radially outer and the radially inner outflow bores (13, 14) while, in accordance with the apparatuses according to Claims 3 to 9, either the conduit (20) as supply conduit, the central filler pipe (16), the second skimmer pipe (22) and possibly the guide disc (17) arranged at a distance from the drum floor (3) are settable in operation on the sieve drum and the radially inner outflow bore (143) is closed and the radially outer outflow bore (13) is opened, or, in accordance with the apparatuses according to Claims 10 to 17, the conduit (20) as skimmer pipe, the filler pipe (23), the central filler pipe (16) with guide disc (17) and the second skimmer pipe (22) are settable in operation on the sieve drum (2) and the radially inner outflow bore (14) is opened and the radially outer outflow bore (13) is closed.

17. Apparatus according to Claims 1 to 16, characterised in that a filter medium is arranged for the retention of the extremely fine particles of the phase kept in suspension, entrained with the through-flowing phase.

**Revendications**

1. Dispositif pour accélérer la réaction entre une phase solide et une phase fluide ou liquide de densités différentes dans un lit fluidisé par centrifugation, dans lequel un espace de réaction est délimité par un cylindre-tamis ou cylindre perforé externe ainsi que, sur ses côtés frontaux, par un fond de tambour correspondant ; l'espace de réaction est entouré à une certaine distance par une enveloppe externe et est fixé à un arbre monté rotatif, dispositif caractérisé en ce que l'espace (8) de réaction est délimité, radialement vers l'intérieur, par un cylindre-tamis (6) ou cylindre perforé intérieur, l'un des fonds de tambour a seulement la forme d'une paroi (5) annulaire, l'espace entouré par l'enveloppe (4) externe est subdivisée en plusieurs tronçons ; il y a, sur un côté frontal de l'espace (8) de réaction, une chambre annulaire (11) et, près de la paroi (5) annulaire, une chambre (12) annulaire d'échappement et, radialement vers l'extérieur, sur le cylindre (7) perforé extérieur de la chambre (8) de réaction, un espace (9) sous pression ; en outre, la chambre annulaire (11) communique avec l'espace (9) sous pression par l'intermédiaire d'un trou (10) de passage et l'espace (8) de réaction communique avec la chambre annulaire (12) d'échappement par l'intermédiaire d'un trou (13) d'échappement ; un tube central (16) de remplissage est prévu pour alimenter l'espace (8) de réaction ; un conduit (20) débouche dans la chambre annulaire (11) et, pour des réactions dans lesquelles la phase traversante a une plus faible densité et la phase devant être retenue en suspension a une plus grande densité, on prévoit un tube (21) pour prélever la phase à plus faible densité dans l'espace (8) de réaction et un autre tube (22) pour prélever la phase à plus grande densité de la chambre annulaire (12) d'échappement.

2. Dispositif selon la revendication 1, caractérisé en ce que l'espace (8) de réaction est délimité par un second cylindre (6) perforé, disposé radialement à l'intérieur au-dessous de l'arête de débordement de la couronne de bordure (5) annulaire.

3. Dispositif selon les revendications 1 et 2, caractérisé en ce que l'acheminement de la phase à plus grande densité dans l'espace (8) de réaction, ménagé entre le cylindre perforé (7) radialement externe et le cylindre perforé (6) radialement interne, s'effectue sur un disque (17) directeur, disposé parallèle et à une certaine distance du fond (3) de tambour et comportant une ouverture (17') formée au centre de ce disque.

4. Dispositif selon les revendications 1 à 3, caractérisé en ce que la chambre annulaire (11) et le conduit (20) d'alimentation constituent un système fermé, dans lequel le conduit d'alimentation est forme par un alésage (18) ménagé dans l'arbre (1) et par des canaux (19) radiaux dirigés vers la chambre (11) annulaire, l'alésage (18) de l'arbre (1) pouvant être relié, par l'intermédiaire d'un joint tournant, à une source de pression.

5. Procédé pour faire fonctionner le dispositif selon les revendications 1 à 4, caractérisé en ce qu'on achemine, par exemple en une fine répartition, vers les deux milieux un milieu gazeux destiné à participer à la réaction.

6. Procédé pour faire fonctionner le dispositif selon les revendications 3 et 5, caractérisé en ce qu'on introduit le milieu gazeux, destiné à réagir dans la chambre (11) annulaire et, par les trous (10) de passage, dans l'espace (9) sous pression.

7. Dispositif selon les revendications 1 à 6, caractérisé en ce qu'on peut acheminer au tambour (2) à double paroi perforée, radialement à l'intérieur du cylindre (6) perforé, radialement interne, et par l'intermédiaire d'un conduit d'alimentation, un milieu de réaction solide ou liquide, éventuellement enrichi en un gaz pour la réaction.

8. Dispositif pour accélérer la réaction entre une phase solide et une phase liquide de densités différentes dans un lit fluidisé, placé dans un champ de centrifugation, dans lequel un espace de réaction est délimité par un cylindre perforé externe ainsi que, sur ses côtés frontaux, par à chaque fois un fond de tambour, et l'espace de réaction est entouré, à une certaine distance, par une enveloppe externe et est fixé à un arbre monté rotatif, dispositif caractérisé en ce que l'espace (8) de réaction est radialement délimité vers l'intérieur par un cylindre (6) perforé interne, l'un des fonds de tambour a seulement la forme d'une paroi (5) annulaire, l'espace entouré par l'enveloppe (4) externe est subdivisé en plusieurs tronçons et il y a, sur un côté frontal de l'espace (8) de réaction, une chambre (11) annulaire ainsi qu'une chambre annulaire (12) d'échappement voisine de la paroi (5) annulaire et, radialement vers l'extérieur, un espace (9) sous pression sur le cylindre (7) perforé externe de l'espace (8) de réaction ; en outre, la chambre annulaire (11) communique avec l'espace (9) sous pression par l'intermédiaire d'un trou (10) de passage et l'espace (8) de réaction, communique avec la chambre annulaire (12) d'échappement par l'intermédiaire d'un trou (13) d'échappement ; on prévoit, pour alimenter l'espace (8) de réaction, un tube central (16) de remplissage ; de plus, un conduit (20) débouche dans la chambre (11) annulaire, et, pour des réactions entre deux milieux, dont la phase traversante a une plus grande densité et la phase à retenir en suspension a une plus faible densité, on prévoit pour alimenter, en la phase de plus grande densité, l'espace (8) de réaction, un tube (23) de remplissage faisant saillie dans l'espace interne du dispositif.

9. Dispositif selon la revendication 8, caractérisé en ce que l'espace (8) de réaction présente, radialement à l'intérieur du trou (10) de passage disposé dans le fond (3) de tambour, un cylindre perforé (7) radialement externe qui sépare l'un de l'autre essentiellement l'espace (9) sous pression et l'espace (8) de réaction.

10. Dispositif selon les revendications 7 et 9, caractérisé en ce que le conduit (20) ; destiné au départ du liquide de réaction, ou de la phase à plus grande densité de la chambre annulaire (11) a la forme d'un tube racleur dont la possibilité de décalage radial permet de régler à volonté, éventuellement de façon intermittente, la chute de pression nécessaire pour que l'espace (8) de réaction soit parcouru par un écoulement dirigé radialement vers l'extérieur.

11. Dispositif selon les revendications 8 et 9 caractérisé en ce que la chambre annulaire (11') constitue un système fermé dans lequel le conduit d'acheminement est formé par un alésage (18) ménagé dans l'arbre (1) et par des canaux (19) partant radialement de la chambre annulaire (11), et en ce que l'espace de centrifugation constitue également un système fermé pouvant être mis en relation avec une surpression.

12. Dispositif selon la revendication 11, caractérisé en ce que l'alésage (18) de l'arbre (1) peut, pour un contre-rinçage du cylindre (7) perforé radialement externe, être mis en communication par l'intermédiaire de joints tournants, avec une source de pression.

13. Procédé pour faire fonctionner le dispositif selon les revendications 8 à 12, caractérisé en ce qu'on achemine vers les deux milieux un milieu gazeux de réaction, sous forme finement divisée.

14. Procédé pour faire fonctionner le dispositif selon la revendication 13, caractérisé en ce qu'on introduit dans le tube (23) de remplissage le milieu gazeux de réaction.

15. Dispositif selon la revendication 13, caractérisé en ce qu'on peut introduire le milieu gazeux de réaction par l'intermédiaire de buses d'injection de gaz, ou d'injecteurs de gaz, répartis à la périphérie de la paroi (4) de tambour, pour alimenter l'espace (8) de réaction, dans une direction radialement vers l'intérieur, à contre-courant de l'écoulement de la phase présentant la plus grande densité.

16. Dispositif selon les revendications 1 à 15, caractérisé en ce que, sur le tambour (2) à double fond perforé ou à tamis, la couronne de bordure (5) annulaire présente aussi bien le trou d'échappement radialement externe que le trou d'échappement radialement interne (13, 14), et, selon les dispositifs correspondant aux revendications 3 à 9, on peut faire fonctionner le conduit (20) comme conduit d'acheminement ou d'alimentation, le tube central (16) de remplissage, le second tube racleur (22) et éventuellement le disque (17) directeur disposé à une certaine distance du fond (3) de tambour sur le tambour à double fond perforé et le trou (14) radialement interne d'échappement peut être fermé et le trou (13) radialement externe d'échappement ouvert ou bien, selon les dispositifs correspondant aux revendications 10 à 17, on peut utiliser le conduit (20) comme tube racleur, le tube (23) de remplissage, le tube central (16) de remplissage avec le disque (17) directeur et le second tube racleur (22) sur le tambour (2) à double paroi perforée et faire fonctionner avec le trou (14) radialement interne d'échappement ouvert et le trou (13) radialement externe d'échappement fermé.

17. Dispositif selon les revendications 1 à 16, caractérisé en ce que, pour retenir les particules les plus fines, entraînées par la phase traversante, de la phase maintenue en suspension, le dispositif comporte un milieu filtrant.

Fig.1

Fig.2

Fig.3

Fig.4